# EUROPÄISCHE PATENTANMELDUNG

(11) **EP 0 727 220 A2**
(43) Veröffentlichungstag der Anmeldung: **21.08.1996**
(21) Anmeldenummer: 96102073.2
(22) Anmeldetag: 13.02.1996
(51) Int. Cl.: A61K 33/14

(54) **Arzneimittel, das als Wirkstoff Magnesiumchlorid enthält**

(30) Priorität: 15.02.1995 DE 19504936
(71) Anmelder: WÖRWAG PHARMA GmbH, D-70499 Stuttgart (DE)
(72) Erfinder: Bauer, Kurt Heinz, D-79112 Freiburg (DE); Wörwag, Fritz, D-70192 Stuttgart (DE)
(74) Vertreter: Weller, Wolfgang, Dr.rer.nat.

(57) **Zusammenfassung**

Ein Arzneimittel, das als Wirkstoff Magnesiumchlorid enthält, liegt in Kombination mit Magnesiumcarbonat in fester Form vor.

## Beschreibung

Die Erfindung betrifft ein Arzneimittel, das als Wirkstoff Magnesiumchlorid und ggf. pharmazeutisch verträgliche Hilfsstoffe enthält.

Ein derartiges Arzneimittel ist aus der EP 0 401 091 A1 bekannt. Das Arzneimittel enthält eine wässerige Lösung von Magnesiumchlorid oder von Magnesiumcarbonat in saurem Millieu mittels organischer Säuren.

Arzneimittel, die als Wirkstoff Magnesiumchlorid enthalten, werden bspw. zur Therapie der Hypomagnesiämie herangezogen (siehe C.-J. Estler "Pharmakologie und Toxikologie", 3. Auflage, Seite 330, Schattauer Verlag).

Die bekannte Therapie besteht in der intravenösen Zufuhr von Magnesiumchlorid.

Magnesiumchlorid in fester Form ist eine hygroskopische Substanz, die aus der Umgebung Wasser anzieht. Durch die Wasseraufnahme quillt Magnesiumchlorid auf und zerfließt nach und nach.

Daher werden bekannte Arzneimittel, die Magnesiumchlorid als Wirkstoff enthalten, ausschließlich in gelöster Form, überwiegend in wässeriger Lösung, und zwar in Form von Infusionslösungen, eingesetzt.

Das Magnesiumchlorid bereitet nicht nur bei der Lagerung oder Halterung aufgrund seiner hygroskopischen Eigenschaften erhebliche Schwierigkeiten, sondern auch bei dessen Verarbeitung. Aufgrund der hygroskopischen Eigenschaften ist es nicht möglich, die üblichen Verfahren zur Herstellung galenisch aufbereiteter Feststoffe wie bspw. Vermischen mit anderen Feststoffen, Pressen in Komprimate durchzuführen, da aufgrund der stark hygroskopischen Eigenschaften pulverförmiges Magnesiumchlorid zum Zusammenbacken neigt, somit die entsprechenden Gerätschaften verklebt. Auch das Herstellen von Komprimaten ist nicht möglich, da die Preßwerkzeuge an hygroskopischem Magnesiumchloridbrei verkleben.

Es ist daher Aufgabe der vorliegenden Erfindung, ein Arzneimittel, das als Wirkstoff Magnesiumchlorid enthält, zu schaffen, das als Feststoff verarbeitbar und als Arzneimittel in fester Form herstellbar ist.

Erfindungsgemäß wird die Aufgabe dadurch gelöst, daß es in Kombination mit Magnesiumcarbonat in fester Form vorliegt.

Untersuchungen haben gezeigt, daß Mischungen aus festem Magnesiumchlorid und festem Magnesiumcarbonat nicht die hygroskopischen Eigenschaften von Magnesiumchlorid zeigen, so daß solche Mischungen nach den gängigen Verfahren zur Herstellung von Feststoffarzneimitteln verarbeitet werden können, ohne daß die Gefahr besteht, daß Werkzeuge, bspw. Preßwerkzeuge oder auch Mischer oder dergleichen, verkleben. Ferner wurde festgestellt, daß solche Feststoffmischungen, nachdem sie einmal verarbeitet sind, ebenfalls nicht mehr die hygroskopischen Eigenschaften von Magnesiumchlorid zeigen, so daß auch die fertiggestellten festen Arzneimittel auf Dauer haltbar sind.

Ein weiterer Vorteil dieser Kombination besteht darin, daß das zugesetzte Mittel Magnesiumcarbonat dieselben Kationen, nämlich Magnesiumionen, wie Magnesiumchlorid enthält, so daß neben den im Arzneimittel wirkenden Kationen Mg²⁺ keine anderen Ionen vorhanden sein müssen. Dies wird als besonderer Vorteil dahingehend angesehen, daß das quasi als "Trocknungsmittel" wirkende Magnesiumcarbonat auch noch zusätzlich das Kation beisteuert, das schon bei dem Wirkstoff Magnesiumchlorid vorhanden ist. Das durch das Magnesiumcarbonat zusätzlich hinzugefügte Carbonat-Anion ist für die therapeutischen Zwecke der Verabreichung von Magnesiumchlorid unschädlich. Bei oraler Verabreichung kann das Carbonat-Anion durch die Magensäure allenfalls durch Chloridionen ersetzt werden, so daß letztendlich nur noch das Anion des Wirkstoffes Magnesiumchlorid, nämlich das Chlorid-Anion, vorliegt.

Die Form der Mischung der Feststoffe kann beliebig gewählt werden, also Pulver, Granulat, Komprimat, Tablette, Dragée oder dergleichen.

Somit wird die Aufgabe vollkommen gelöst.

In einer vorteilhaften Ausgestaltung der Erfindung liegt das Magnesiumcarbonat als wasserfreies Magnesiumcarbonat, als leicht basisches Magnesiumcarbonat und/oder als schweres basisches Magnesiumcarbonat oder in einer anderen Form vor.

Diese Auswahl hat den Vorteil, daß das Magnesiumcarbonat in unterschiedlich löslichen Formen zugesetzt werden kann, die auch eine abgestufte "Trocknungswirkung" aufweisen, so daß dem Galeniker eine große Variationsbreite an Magnesiumcarbonatzusätzen zur Verfügung steht. Leichtes basisches Magnesiumcarbonat und schweres basisches Magnesiumcarbonat sind auf dem Gebiet der Medizin eingesetzte Magnesiumcarbonatverbindungen, die neben Magnesiumcarbonat noch Magnesiumhydroxyd in einem bestimmten Anteil enthalten.

In einer weiteren Ausgestaltung der Erfindung liegt das Magnesiumchlorid als wasserfreies Magnesiumchlorid, als Magnesiumchlorid-6-Wasser oder als Magnesiumchlorid-7-Wasser oder in einer anderen Form vor.

Diese Auswahl gibt nun andererseits gegenüber dem zuvor erwähnten Magnesiumcarbonat als Verarbeitungspartner die entsprechende breite Bandbreite an Wirkstoff, um für den jeweils gewünschten Einsatzzweck die jeweils optimale Feststoffmischung vorliegen zu haben.

Dadurch wird dem Galeniker die Möglichkeit eröffnet, in Abhängigkeit davon, ob es sich um langwierige Verarbeitungsverfahren handelt, bei denen das Magnesiumchlorid sehr lange mit der Luftfeuchtigkeit in Berührung treten kann, oder ob es sich nur um kurzzeitige Misch- und Preßverfahren handelt, flexibel darauf zu reagieren, und er kann die dafür optimale Mischung an Magnesiumchlorid und Magnesiumcarbonat auswählen. Diese Mischung wird auch in Abhängigkeit der endfertigen Galenik auszuwählen sein, d.h., ob das in fester Form vorliegende Endprodukt abschließend noch beschichtet wird und wie die Durchlässigkeit gegenüber Feuchtigkeit von solchen Beschichtungen ist. Werden Beschichtungen eingesetzt, die nur wenig oder überhaupt nicht wasserdurchlässig sind, so reicht es, so viel an Magnesiumcarbonat zuzusetzen, daß während der Verarbeitung keine nennenswerte Feuchtigkeit aufgenommen werden kann.

Je nach den zuvor erwähnten Umständen liegt das Gewichtsverhältnis zwischen Magnesiumchlorid und Magnesiumcarbonat zwischen 1 : 100 und 100 : 1. Dabei liegt es vorzugsweise zwischen 1 : 1 und 10 : 1 und höchst vorzugsweise zwischen 4 : 1 und 8 : 1. In diesen Mengenverhältnissen sind die gängigen Verarbeitungs- und Konfektionsverfahren problemlos durchzuführen. Wird das erfindungsgemäße Arzneimittel in eine wasserundurchlässige Umhüllung verpackt, so daß auch auf Dauer keine Feuchtigkeit an den Feststoff Magnesiumchlorid herangebracht werden kann, sind geringe Mengen an Magnesiumcarbonat ausreichend. Es reichen solche geringen Mengen aus, die ein Verarbeiten (Mischen, Komprimieren) erlauben, ohne daß die Werkzeuge (z.B. Stempel) dafür verkleben. Je mehr das endfertige Produkt der Feuchtigkeit ausgesetzt ist, insbesondere auch der Luftfeuchtigkeit, desto höher ist der Anteil am "Trocknungsmittel" Magnesiumcarbonat.

Bevorzugt sind solche festen Arzneimittel, die als Komprimat vorliegen, und dies ist ggf. äußerlich mit einer Lackierung versehen.

Als Komprimat liegt das Arzneimittel als kompakter Körper vor, dessen mit der Umgebung in Kontakt stehende Oberfläche zur Masse relativ gering ist.

Eine Lackierung hat nun den Vorteil, daß durch eine äußere Schutzschicht das Eindringen von Feuchtigkeit in den Kern der Magnesiumchlorid/Magnesiumcarbonatmischung erschwert oder vollständig gehindert ist, so daß auch auf Dauer eine große Lagerstabilität erzielt werden kann. Dadurch können solche Tabletten bspw. in Tablettenröhrchen verpackt werden und müssen nicht nochmals in zusätzlichen Schutzverpackung wie bspw. Blisterverpackungen konfektioniert werden.

In einer weiteren Ausgestaltung der Erfindung weist das Arzneimittel über der Lackierung noch eine Acrylharzschicht auf.

Diese Maßnahme hat nun den Vorteil, daß eine für die orale Verabreichung besonders günstige Außenfläche geschaffen wird, die darüber hinaus durch entsprechende Auswahl des Acrylharzes einfach mit einer wasserundurchlässigen äußersten Schicht versehen werden kann.

Bei der Verarbeitung der Bestandteile Magnesiumchlorid und Magnesiumcarbonat können die üblichen pharmazeutisch verträglichen Trägersubstanzen und Verarbeitungshilfsstoffe wie Cellulose, Öle und dergleichen mit verarbeitet werden.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der Erfindung zu verlassen. Die Erfindung wird nachfolgend anhand eines ausgewählten Ausführungsbeispiels näher beschrieben und erläutert.

### Beispiel 1:

Es werden die nachfolgenden Rohstoffe Ziffer 1. - 7. eingewogen, über ein Sieb mit 1,5 mm Maschenweite gesiebt und in einem "Rhönrad"-Mischer 15 Minuten bei 25 Umdrehungen pro Minute gemischt.

### Rohstoffmischung

| | | |
|---|---|---|
| 1. | Magnesiumchlorid-6-hydrat | 20,00 kg |
| 2. | Magnesiumcarbonat, schwer basisch | 3,00 kg |
| 3. | Rizinus, Öl, hydriert, Cutina HR | 4,00 kg |
| 4. | Polyvinyl-Polypyrrolidon (PVPP), kreuzvernetzt, unlöslich-Polyplasdone XL (Crospovidone NF) | 2,50 kg |
| 5. | Siliciumdioxid, hochdisp. CAB-O-SIL M-5 | 0,66 kg |
| 6. | Cellulose, Carboxymethyl-Natrium, Ac-Di-Sol Typ SD 711 NF (Croscarmelose Sodium) | 1,32 kg |
| 7. | Cellulose, Sanacel 150 | 1,02 kg |

Die preßfertige Mischung wird auf einer Rundläufer-Tablettiermaschine zu einem Oblong 10 mm x 25 mm, ohne Bruchrille, mit einem Gewicht von 1.625 mg gepreßt.

Die gepreßten Kerne werden anschließend mit folgender Lösung lackiert, bis das Gewicht 1.650 mg beträgt.

### Lackierlösung

| | |
|---|---|
| Cellulose, Hydroxypropyl, Klucel LF | 0,500 kg |
| Ethanol, unvergällt, 96 % Mindestalkoholgehalt, Sorte 410 | 4,500 kg |

Nach dem Aufbringen der Filmlösung wird etwa für zwei Stunden nachgetrocknet.

Derartig hergestellte Tabletten sind an der Umgebung für mehrere Monate haltbar, ohne daß ein durch Feuchtigkeitsaufnahme verursachtes Aufquellen oder sonstige Veränderungen beobachtet werden. Auch an den Verarbeitungsgeräten wurden keine Verklebungen oder feuchte Rückstände festgestellt.

### Beispiel 2:

Es werden gepreßte Kerne entsprechend Beispiel 1 hergestellt, die jedoch nicht mit einer Lackierlösung versehen werden.

Auch die gepreßten "nackten" Kerne sind mehrere Wochen an der Umgebung ohne Anzeichen einer Wasseraufnahme durch Aufquellen lagerbar.

### Beispiel 3:

Ein nach der Verfahrensvorschrift von Beispiel 1 hergestelltes lackiertes Oblong wird noch mit einem Acrylharz (Eudragit E100), das aus einer Mischung von Dimethylaminoethylmethacrylat und einem Methacrylsäureester mit einem mittleren Molekulargewicht von 150.000 zusammengesetzt ist, oberflächlich besprüht.

Die so erhaltene Filmtablette kann mit Wasser benetzt werden, ohne daß ein Aufquellen als Anzeichen von Wasseraufnahme beobachtet wird.

### Beispiel 4:

Es wird ein Oblong mit 10 mm x 25 mm entsprechend der Vorschrift von Beispiel 1 hergestellt, mit der Ausnahme, daß der Gehalt an Magnesiumcarbonat an der Rohrstoffmischung 6,000 kg ist.

Ein solcher Rohling zeigt bei freiem Liegen an der Umgebung selbst nach mehreren Wochen keine Anzeichen von Wasseraufnahme durch Aufquellen.

Dies gilt gleichermaßen für Bruchstellen an einem aufgebrochenen Rohling.

### Beispiel 5:

Es werden Kerne entsprechend der Vorschrift von Beispiel 1 hergestellt, jedoch nur mit 1,0 kg und mit 0,50 kg Magnesiumcarbonat. Auch hier zeigen die Verarbeitungsgerätschaften, insbesondere die Tablettiermaschine, keine Verklebungen durch eine feuchtgewordene Rohstoffmischung.

## Patentansprüche

1. Arzneimittel, das als Wirkstoff Magnesiumchlorid und ggf. pharmazeutisch verträgliche Hilfsstoffe enthält, dadurch gekennzeichnet, daß es in Kombination mit Magnesiumcarbonat in fester Form vorliegt.

2. Arzneimittel nach Anspruch 1, dadurch gekennzeichnet, daß das Magnesiumcarbonat als wasserfreies Magnesiumcarbonat, als leichtes basisches Magnesiumcarbonat und/oder als schweres basisches Magnesiumcarbonat oder in einer anderen Form vorliegt.

3. Arzneimittel nach Anspruch 1 oder 2, dadurch gekennzeichnet, daß das Magnesiumchlorid als wasserfreies Magnesiumchlorid, als Magnesiumchlorid-6-Wasser oder als Magnesiumchlorid-7-Wasser oder in einer anderen Form vorliegt.

4. Arzneimittel nach einem der Ansprüche 1 bis 3, dadurch gekennzeichnet, daß das Gewichtsverhältnis zwischen Magnesiumchlorid und Magnesiumcarbonat zwischen 1 : 100 und 100 : 1 liegt.

5. Arzneimittel nach Anspruch 4, dadurch gekennzeichnet, daß das Gewichtsverhältnis zwischen Magnesiumchlorid und Magnesiumcarbonat zwischen 1 : 1 und 10 : 1 liegt.

6. Arzneimittel nach Anspruch 4, dadurch gekennzeichnet, daß das Gewichtsverhältnis zwischen Magnesiumchlorid und Magnesiumcarbonat zwischen 4 : 1 und 8 : 1 liegt.

7. Arzneimittel nach einem der Ansprüche 1 bis 6, dadurch gekennzeichnet, daß es als Komprimat vorliegt.

8. Arzneimittel nach einem der Ansprüche 1 bis 7, dadurch gekennzeichnet, daß es äußerlich mit einer Lackierung versehen ist.

9. Arzneimittel nach Anspruch 8, dadurch gekennzeichnet, daß es über der Lackierung noch eine Acrylharzschicht aufweist.

10. Arzneimittel nach einem der Ansprüche 1 bis 9, dadurch gekennzeichnet, daß neben Magnesiumchlorid, Magnesiumcarbonat und den pharmazeutisch verträglichen Hilfsstoffen, auch Verarbeitungshilfen zur Herstellung von Komprimaten enthalten sind.
